# EUROPÄISCHE PATENTSCHRIFT

(11) **EP 1 754 501 B1**
(45) Veröffentlichungstag und Bekanntmachung des Hinweises auf die Patenterteilung: **04.11.2015**
(21) Anmeldenummer: 06016984.4
(22) Anmeldetag: 15.08.2006
(51) Int. Cl.: A61M 25/00, A61M 39/26, A61F 5/44

(54) **Katheterventil**
valve for catheter
valve pour cathéter

(30) Priorität: 16.08.2005 DE 102005038651
(43) Veröffentlichungstag der Anmeldung: 21.02.2007
(73) Patentinhaber: Pfleiderer, Klaus, 60433 Frankfurt am Main (DE)
(72) Erfinder: Pfleiderer, Klaus, 60433 Frankfurt am Main (DE)
(74) Vertreter: Patentanwälte Olbricht Buchhold Keulertz

(56) Entgegenhaltungen:
- EP-A2- 0 873 763
- WO-A-03/018105
- WO-A2-01/62315
- CH-A5- 684 165
- DE-A1- 19 718 582
- DE-C1- 19 915 342
- US-A- 5 269 771
- US-A- 5 297 776
- US-A- 6 165 168

## Beschreibung

Die vorliegend Erfindung befaßt sich mit einem Katheterventil mit einem Gehäuse, das eine an einen Urinableitungskatheter anschließbare Eintrittsöffnung, eine mit einem Anschlußstück eines Auffangbehälters dicht verbindbare Austrittsöffnung und einen Ventilsitz aufweist, der mit einem in Schließrichtung federnd vorbelasteten, axial verschieblichen Ventilkörper zusammenwirkt, welcher mittels eines mit ihm in Eingriff stehenden Griffstücks manuell in Öffnungsrichtung verschiebbar ist und einen sich durch die Dichtfläche des Ventilsitzes hindurch zur Austrittsöffnung erstreckenden Vorsprung hat.

Ein derartiges Katheterventil ist z. B. aus der EP 0 873 763 A2 bekannt. Es hat einen axial verschieblichen, ggf. am Ende kegelförmigen Ventilkörper, der mit einem die Austrittsöffnung des Gehäuses umgebenden ggf. konischen Ventilsitz zusammenwirkt. Das Aufstecken eines zum Auffangbehälter führenden Schlauchs auf einen die Austrittsöffnung enthaltenden Gehäuseansatz beeinflusst nicht die Stellung des Ventilkörpers.

Ein weiteres, in der Funktion vergleichbares Katheterventil ist in der CH 684 165 A5 beschrieben. In diesem Fall befindet sich in einer elastisch biegsamen Leitung ein Ventilkörper, der mittels eines quer zur Längsrichtung der Leitung verschiedlich geführten Drückers durch Biegung der Leitung in eine Öffnungsstellung zu bringen ist. Ebenfalls ähnlich funktioniert ein aus der DE 197 18 582 A1 bekanntes Katheterventil, bei dem ein elastischer Schlauchabschnitt durch axialen Druck mittels eines hülsenförmigen Griffstücks zu einer Schlaufe verformt und zusammengequetscht wird.

Die DE 199 15 342 C1 und die WO 01/62 315 A1 beschreiben urologische Katheterventile mit einem steuerbaren Ventilkörper, der nicht durch eine Federkraft in Schließrichtung vorbelastet und somit nur manuell umschaltbar ist.

Weitere Ventile sind im Zusammenhand mit Injektions- oder Infusionsleitungen bekannt. Die US-A-5 269 771 beschreibt z. B. ein Zwischenventil, das durch das Einführen eines Anschlussstücks der Injektion- oder Infusionseinrichtung in die Eintrittsöffnung in Öffnungsstellung umschaltbar ist, allerdings nur, wenn auch ein Griffstück in eine Öffnungsstellung verschoben worden ist. Die WO 03/018105 A1 zeigt ein an eine Injektionseinrichtung anzuschließendes Ventil, das einen Rückfluss verhindern soll. Eine manuelle Umsteuerung mittels eines Griffstücks ist dabei nicht vorgesehen. Die US-A-5 297 776 beschreibt eine Kupplungsvorrichtung mit einer Sicherungseinrichtung gegen Kontaminationen. Dabei muss ein Venteilkörper zur Umschaltung mittels eines Griffstücks nicht nur axial verschoben sondern auch gedreht werden.

Bei den bekannten Katheterventilen kann die Ableitung von Urin kontinuierlich in eine Auffangvorrichtung, z. B. in einen Urinbeutel, oder intermittierend vom Patienten betätigt, beispielsweise in ein Abflußbecken, erfolgen. Im letzteren Fall ist der Katheter so ausgelegt, daß der Patient das mit dem Ende des Katheters abdichtend verbundene Katheterventil über oder in einen Auffangbehälter hält und das Ventil manuell öffnet. Für eine kontinuierliche Ableitung wird die Austrittsöffnung des Katheterventils beispielsweise mit einem Urinbeutel verbunden. Da die handelsüblichen Urinbeutel unterschiedliche Anschlußstücke aufweisen, ist für den Anschluß eines Katheterventils jedoch noch ein Adapterstück erforderlich. Wenn der Urinbeutel mittels eines Adapterstücks an ein übliches Katheterventil angeschlossen werden soll, wird das Katheterventil für eine permanente Ableitung manuell in eine Öffnungsstellung gebracht. Als Vorrichtung für eine dauerhafte Öffnung offenbart z. B. die EP 0 873 763 A2 einen um ein längliches Gehäuse verlaufenden Ring oder eine Rastnut, mit deren Hilfe ein Schieber zum Öffnen des Ventils festgestellt werden kann.

Es hat sich jedoch während des Alltagsbetriebs z. B. in Krankenhäusern oder anderen medizinischen Einrichtungen gezeigt, daß oft nach dem Anschließen eines Urinbeutels das Öffnen des Katheterventils vergessen wird. Als Folge dessen findet keine Ableitung der Körperflüssigkeit statt, was für den Patienten bei längeren Zeiträumen kritisch sein kann. Als typischer Fall sei hier auf den Anschluß von Urinbeuteln während der Nacht hingewiesen. Wird der Urinbeutel angeschlossen, ohne jedoch das Katheterventil zu öffnen, kann bis zum nächsten Morgen kein Urin abfließen und der Stau starke Schmerzen verursachen. Ein weiterer Nachteil der bekannten Lösungen besteht darin, daß ein Abtrennen s.B. eines Urinbeutels - sei es beispielsweise aufgrund einer unachtsamen Bewegung des Patienten oder einer Unaufmerksamkeit des Pflegepersonals beim Auswechseln - bei geöffneter Ventilstellung zu einer hygienisch problematischen Verschmutzung oder gar Kontaminierung des Umgebungsbereichs führen kann. Dieser Mangel bleibt, auch dann bestehen, wenn das Katheterventil, wie z. B. im Fall der DE 199 15 342 C1, so konstruiert ist, dass es in der geöffneten Stellung eingerastet werden muss, bevor das mit dem Auffangbehälter verbundene Anschlußstück an das Katheterventil angeschlossen werden kann.

Die Aufgabe der vorliegenden Erfindung besteht darin, ein Katheterventil zu schaffen, das eine potentielle Fehlbedienung ausschließt und das den für den Anschluß eines Auffangbehälters, z. B. eines Urinbeutels, erforderlichen Aufwand auf ein Minimum reduziert.

Erfindungsgemäß wird die Aufgabe durch ein Katheterventil der eingangs bezeichneten Art gelöst, bei dem der Versprung des Ventilkörpers im Querschnitt um so viel kleiner als der Querschnitt der Austrittsöffnung ist, dass ein axialer Strömungskanal in das Anschlußstück frei bleibt, aber ausreichend groß, dass das in die Austrittsöffnung einführbare Anschlußstück stellenweise gegen den Vorsprung stößt und dadurch der Ventilkörper vom Ventilsitz weg in eine Öffnungsstellung zurückdrängbar ist.

Der Vorteil des erfindungsgemäßen Katheterventils mit zweifacher Öffnungsmöglichkeit besteht darin, daß nicht nur durch Betätigung des Griffstücks, sondern auch durch den Anschluß eines Urinbeutels das Katheterventil geöffnet wird. Der bisher notwendige, zusätzliche Handgriff des Pflegepersonals zum Öffnen des Ventils nach dem Anschluß eines Urinbeutels entfällt, und damit auch das Risiko einer Fehlbedienung durch Unterlassen des Öffnens.

Besonders bevorzugt ist eine Ausführungsform, bei der die Austrittsöffnung eine anpassungsfähige Auskleidung zur dichten Verbindung mit Anschlußstücken unterschiedlicher Form und Größe hat. Dadurch ist es möglich, eine Vielzahl handelsüblicher Urinbeutel mit ihrem jeweiligen Anschlußstück direkt mit der Austrittsöffnung des Katheterventils zu verbinden. Sowohl der Vorgang des Anschließens eines Adapterstücks als auch das Bevorraten von Adapterstücken können normalerweise entfallen. Als Austrittsöffnung wird im Rahmen dieser Anmeldung der Auslaß hinter dem Ventilsitz bezeichnet, auch wenn die Austrittsöffnung im Einzelfall eher die Form eines Austrittskanals hat.

Die anpassungsfähige Auskleidung der Austrittsöffnung hat vorzugsweise einen sich von außen nach innen verengenden lichten Querschnitt, um insbesondere Urinbeutel oder Adapter mit konischen Anschlußstücken verwenden zu können, die zur einfachen Herstellung einer dichten Leitungsverbindung besonders geeignet sind. Die Austrittsöffnung bildet einen sogenannten weiblichen Adaptionskonus.

Weiterhin bevorzugt ist eine Ausführungsform, bei welcher der Ventilkörper einen sich durch die Dichtfläche des Ventilsitzes in die Austrittsöffnung erstreckenden Vorsprung hat, gegen den das Anschlußstück beim Einführen in die Austrittsöffnung stößt. Dadurch ist auf einfache Weise gewährleistet, daß sich das Ventil in einer offenen Stellung befindet, sobald das Anschlußstück in die Austrittsöffnung eingeführt ist. Gleichzeitig übernimmt der Vorsprung auch eine gewisse axiale Führung des Ventilkörpers.

In zweckmäßiger Ausgestaltung der Erfindung ist der Vorsprung im Querschnitt um so viel kleiner als der Querschnitt der Austrittsöffnung, daß ein axialer Strömungskanal frei bleibt, aber ausreichend groß, daß ein Anschlußstück stellenweise dagegen anstoßen und den Ventilkörper vom Ventilsitz abheben kann, damit die Flüssigkeit in das Anschlußstück fließen kann.

Zu diesem Zweck kann das freie Ende des Vorsprungs mit Aussparungen oder Kanälen versehen sein, die einen mit dem axialen Strömungskanal in Verbindung stehenden radialen Strömungskanal bilden, so daß die Körperflüssigkeit neben der oder den Berührungsstellen in das Anschlußstück gelangt.

Die Austrittsöffnung und der Vorsprung haben vorzugsweise einen zueinander passenden, runden Querschnitt und der Vorsprung hat eine oder mehrere rillenförmige, sich axial erstreckende Ausnehmungen. Die runde Form ermöglicht einen zuverlässig dichten Sitz und vermeidet scharfe Kanten, die zu Verschleiß führen könnten. Die Rillen gewährleisten den Abfluß der Körperflüssigkeit, auch wenn sich der Vorsprung in der Öffnungsstellung noch hinter dem Ventilsitz im Austrittskanal befindet und ein Anschlußstück den Vorsprung teilweise übergreift.

In einer anderen Ausführungsvariante ist der Vorsprung durch einen zylindrischen Körper mit einem sich zu seiner Mittelachse hin z. B. konisch oder ballig verjüngenden Ende gebildet. Durch das sich verjüngende Ende erfolgt beim Einführen eines Anschlußstücks automatisch eine Zentrierung von Anschlußstück und Ventilkörper, da sich das freie Ende des Vorsprungs auf die Mitte der runden lichten Öffnung eines Anschlußstücks einstellt.

Eine besonders einfache Ausführungsform gewinnt man, wenn der Ventilkörper ein länglicher, in axialer Richtung verschieblichen Hohlkörpers ist, dessen auf Seiten der Eintrittsöffnung gelegene Stirnseite offen ist, dessen andere Stirnseite geschlossen ist und mit dem Ventilsitz zusammenwirkt und der in Strömungsrichtung vor dem Ventilsitz seitliche Öffnungen hat. Dies ermöglicht eine kostengünstige Herstellung und eine einfache Montage, da die Zahl der zusammenzufügenden Einzelteile auf ein Minimum reduziert ist.

Zur Weiterbildung der Erfindung ist vorgesehen, daß der Ventilkörper axial beweglich in einem Hohlraum gehalten ist, der Teil des Strömungskanals durch das Gehäuse ist und durch eine Hülse aus elastischem Material gebildet ist, die sich innerhalb des Gehäuses erstreckt und an dessen Enden in dichter Verbindung mit der Ein- und Austrittsöffnung befestigt ist. Durch die Hülse aus elastischem Material ist eine zuverlässige Abdichtung gewährleistet.

Zweckmäßigerweise ist die Hülse axial geteilt und weist einen Kompressionsbalg auf, der sich zwischen der Eintrittsöffnung und einer äußeren Rastnut des Ventilkörpers erstreckt, sowie eine Dichtungstülle, die sich zwischen der Rastnut und dem Ventilsitz oder der Austrittsöffnung erstreckt. Somit können für die verschiedenen Bereiche auch verschiedene Materialien eingesetzt werden, die an ihre jeweilige Aufgabe optimal angepaßt werden können.

In der praktischen Ausführung ist die Dichtungstülle einstükkig mit der Auskleidung der Austrittsöffnung ausgebildet, welche deren anpassungsfähigen Öffnungsrand bildet, um eine saubere Abdichtung zu gewährleisten.

Für eine kostengünstige Ausführungsform bietet es sich an, daß die federnde Vorbelastung des Ventilkörpers durch den Kompressionsbalg und/oder durch die Dichtungstülle erzeugt wird, so daß hierfür kein zusätzliches Federglied gebraucht wird.

Bei einer alternativen erfindungsgemäßen Ausführungsform ist anstelle der Hülse aus elastischem Material vorgesehen, daß der hohlzylindrische Ventilkörper jeweils mittels gleitender Dichtungen axial verschieblich an der Innenfläche der zylindrischen Gehäusewand und an der zylindrischen Außenfläche eines sich axial von der Eintrittsöffnung in den von der Gehäusewand umgrenzten inneren Hohlraum hinein erstreckenden Kanals geführt und von einer in dem Ringraum zwischen der Gehäusewand und dem Kanal aufgenommenen Feder in Schließrichtung vorbelastet ist. Dabei ist vorzugsweise die Dichtung zwischen dem Ventilkörper und der Gehäusewand mit Bezug auf die Strömungsrichtung vor den seitlichen Öffnungen und die Dichtung zwischen dem sich an die Eintrittsöffnung anschließenden Kanal und dem Ventilkörper an dessen in Strömungsrichtung vorderen Ende angeordnet, und das mit dem Ventilkörper verbundene Griffstück ragt im axialen Bereich zwischen den beiden ringförmigen Dichtungen durch ein Loch in der Gehäusewand nach außen.

Die Erfindung in ihren verschiedenen Ausführungsformen kann noch dahingehend weitergebildet werden, daß durch die Bewegung des Griffstücks bei der Verschiebung des Ventilkörpers mittels des Anschlußstücks in eine Öffnungsstellung auf der Außenseite des Gehäuses z.B. ein farbiges oder leuchtendes Signalelement freilegbar ist. Dadurch ist eine visuelle Kontrolle möglich, ob sich das Anschlußstück auch wirklich in einer dicht sitzenden Anschlußstellung befindet, in der es den Ventilkörper ausreichend weit vom Ventilsitz weg in eine Öffnungsstellung zurückgedrängt hat.

Nachfolgend wird anhand der beigefügten Zeichnung näher auf Ausführungsbeispiele der Erfindung eingegangen. Es zeigen:
- Fig. 1: einen Längsschnitt eines erfindungsgemäßen Katheterventils;
- Fig. 2: einen Längsschnitt (Ausschnitt) des Katheterventils nach Fig. 1 und eines Anschlußstücks eines Auffangbehälters, das in die Austrittsöffnung des Katheterventils eingeführt ist;
- Fig. 3: einen Längsschnitt (Ausschnitt) eines Katheterventils in geschlossenem Zustand;
- Fig. 4: einen Längsschnitt (Ausschnitt) des Katheterventils nach Fig. 3 in geöffnetem Zustand;
- Fig. 5: eine Aufsicht auf einen Vorsprung am Ventilkörper des Katheterventils nach Figs. 3 und 4;
- Fig. 6: einen Teil-Längsschnitt des Katheterventils mit einem Vorsprung mit rundem Ende am Ventilkörper;
- Fig. 7: einen Teil-Längsschnitt einer weiteren Ausführungsform eines Vorsprungs am Ventilkörper des Katheterventils in Anschlußstellung mit einem eingeführten Anschlußstück;
- Fig. 8: eine Aufsicht des Vorsprungs nach Fig. 7;
- Fig. 9: eine weitere Ausführungsform eines Vorsprungs am Ventilkörper des Katheterventils in Anschlußstellung mit einem eingeführten Anschlußstück;
- Fig. 10: eine Aufsicht des Vorsprungs nach Fig. 9;
- Fig. 11: eine Seitenansicht des Katheterventils mit einem Griffstück und einem Schema zum Bewegungs- und Arretierungsvorgang des Griffstücks und
- Fig. 12: einen Längsschnitt durch eine weitere Ausführungsvariante des neuen Katheterventils.

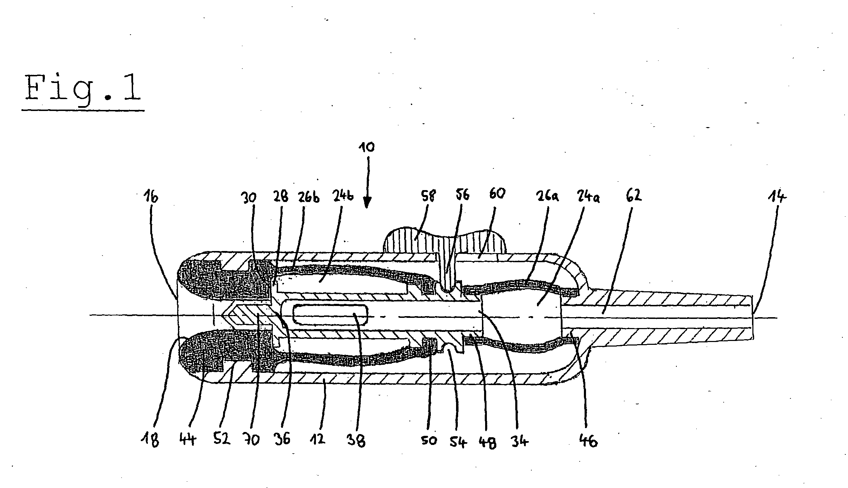

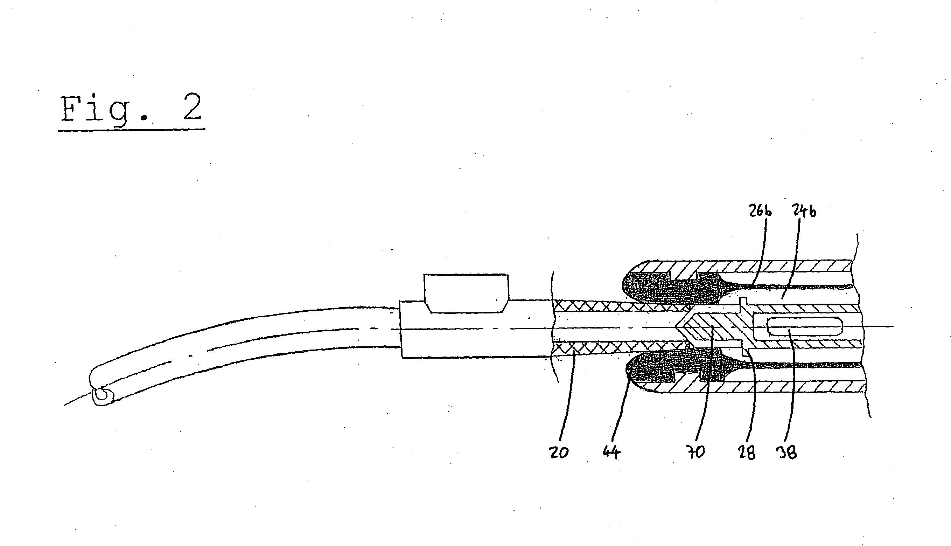

In Fig. 1 ist in einem Längsschnitt ein erfindungsgemäßes Katheterventil 10 gezeigt, das ein Gehäuse 12 mit einer Eintrittsöffnung 14, an die ein nicht dargestellter Ableitungskatheter angeschlossen werden kann, und einer Austrittsöffnung 16 aufweist, die einen anpassungsfähigen Öffnungsrand 18 mit einem sich von außen nach innen verengenden lichten Querschnitt hat und die mit einem Anschlußstück 20 (Fig. 2) eines nicht gezeigten Auffangbehälters zu verbinden ist.

Das Gehäuse 12 enthält eine zweiteilige Hülse 26a, 26b aus elastischem Material, z.B. Silikon oder einem thermoplastischen Elastomer, die einen sich zwischen der Eintrittsöffnung 14 und der Austrittsöffnung 16 erstreckenden Hohlraum 24a, 24b begrenzt, der Teil des Strömungskanals durch das Gehäuse 12 ist. In dem Hohlraum 24a, 24b ist ein Ventilkörper 28 angeordnet, der mit einem Ventilsitz 30 zusammenwirkt und durch einen axial beweglich gehaltenen Hohlkörper gebildet ist, der eine auf Seiten der Eintrittsöffnung 14 gelegene, offene Stirnseite 34, am anderen Ende eine geschlossene Stirnseite 36 und seitliche, in Strömungsrichtung vor dieser Stirnseite 36 gelegene Öffnungen 38 hat, die als Umlenkschlitze dienen.

Die Hülse 26a, 26b ist axial geteilt und weist einen Kompressionsbalg 26a und eine Dichtungstülle 26b auf, die einstückig mit einer den anpassungsfähigen Öffnungsrand 18 der Austrittsöffnung 16 bildenden Auskleidung 44 ausgebildet ist.

Der Ventilkörper 28 ist durch den Kompressionsbalg 26a in Schließrichtung federnd vorbelastet. Zur zusätzlichen Federwirkung kann auch die Dichtungstülle 26b herangezogen werden. Vorstellbar ist weiterhin, daß die Federwirkung nur von der Dichtungstülle 26b oder auch von einer zusätzlichen Feder ausübbar ist. Die Federwirkung ist durch entsprechende Auslegung des Materials, der Materialstärke und der Form des Kompressionsbalgs 26a und/oder der Dichtungstülle 26b einstellbar. Der Kompressionsbalg 26a ist abdichtend an einem auf der Innenseite des Gehäuses 12 befindlichen Vorsprung 46 der Eintrittsöffnung 14 befestigt und auf der anderen Seite mit einem gegenüberliegenden Vorsprung 48 des Ventilkörpers 28 abdichtend verbunden. Die Dichtungstülle 26b ist an ihrem in Fließrichtung stromaufwärts gelegenen Ende mit dem Ventilkörper 28 mittels einer Nut 50 abdichtend verbunden. Die Auskleidung 44, mit der die Dichtungstülle 26b an ihrem anderen, in Fließrichtung stromabwärts gelegenen Ende einstückig ausgebildet ist, ist mit dem Gehäuse 12 mittels eines umlaufenden, innenseitigen Vorsprungs 52 verbunden. Zwischen der Nut 50 und dem Vorsprung 48 ist eine Rastnut 54 angeordnet, in die ein Vorsprung 56 eines Griffstücks 58 eingreift. Das Griffstück 58 ist in einer Führungsnut 60 des Gehäuses 12 verschiebbar.

In Fig. 2 ist ein in die Austrittsöffnung 16 eingeführtes Anschlußstück 20 eines nicht dargestellten Auffangbehälters gezeigt. Durch das Einführen des Anschlußstücks 20 in die Austrittsöffnung 16 ist der Ventilkörper 28 von dem Ventilsitz 30 in eine Öffnungsstellung zurückgedrängt. Das Anschlußstück 20 ist mit Hilfe des anpassungsfähigen Öffnungsrands 18 der Auskleidung 44 abdichtend mit dieser verbunden.

Bei Anschluß eines Ableitungskatheters an die Eintrittsöffnung 14 fließt die Körperflüssigkeit durch einen Eintrittskanal 62 in den Hohlraum 24a, der durch den Kompressionsbalg 26a begrenzt wird, und von dort weiter in den hohlen Ventilkörper 28. Über die seitlichen Öffnungsschlitze 38 gelangt die Flüssigkeit in einen von der Dichtungstülle 26b gebildeten Hohlraum 24b, der zur Austrittsöffnung 16 hin in geschlossenem Zustand durch den Ventilkörper 28 abgedichtet ist. In geöffnetem Zustand des Katheterventils 10 gelangt die Flüssigkeit aus dem Hohlraum 24b in das Anschlußstück 20, um von dort in den nicht gezeigten Auffangbehälter abzufließen.

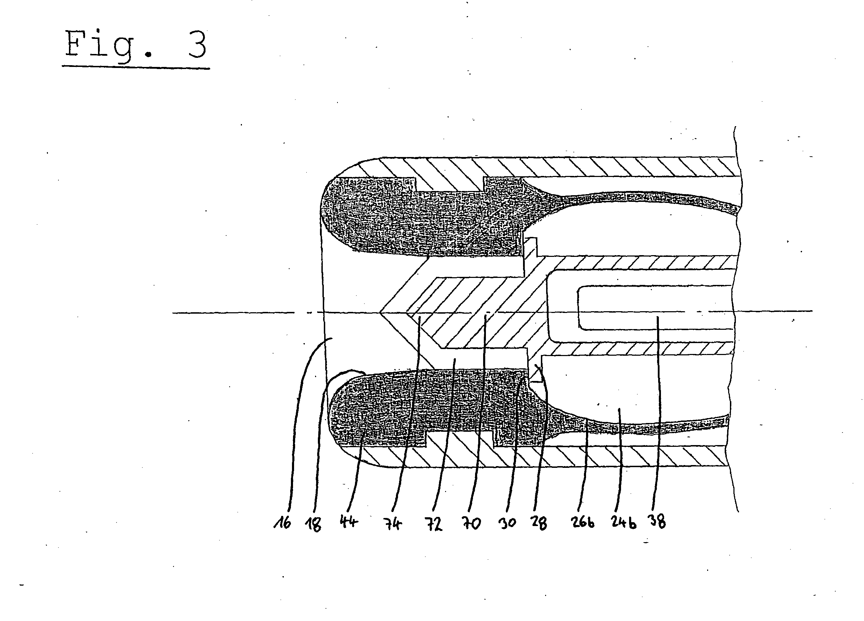

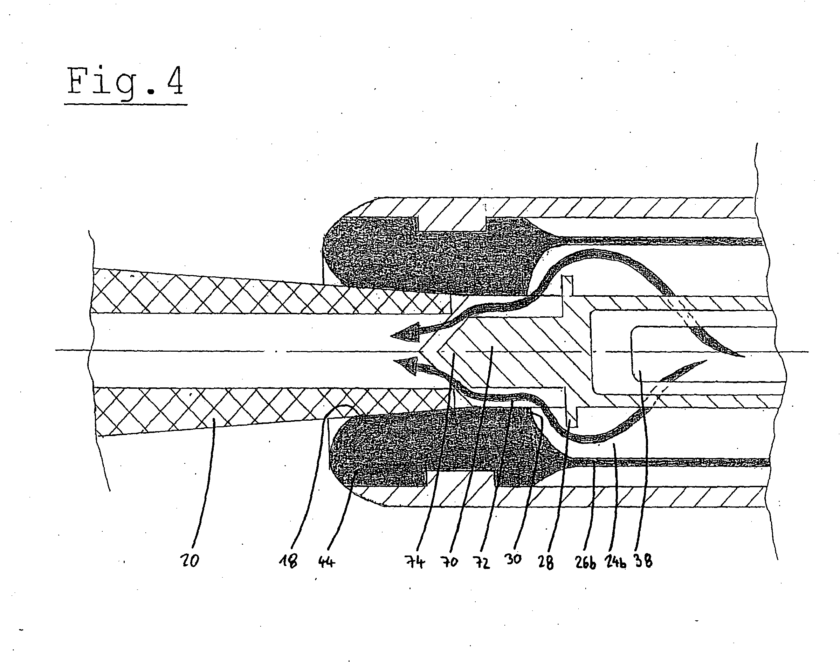

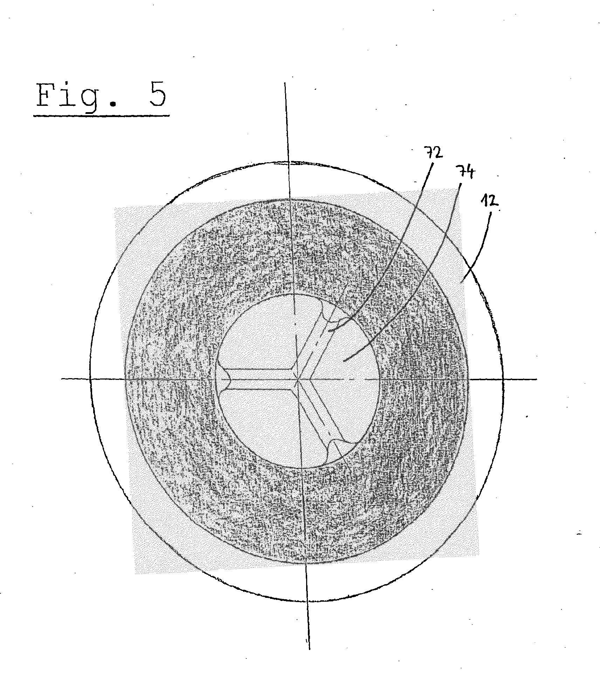

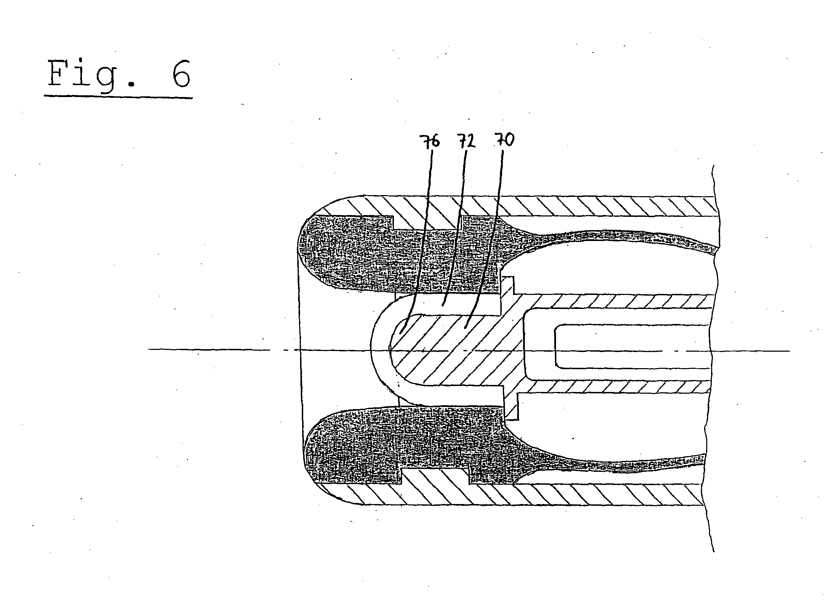

In Fig. 3 ist ein Ventilkörper 28 gezeigt, der einen sich durch die Dichtfläche des Ventilsitzes 30 hindurch zur Austrittsöffnung 16 erstreckenden Vorsprung 70 hat, gegen den das Anschlußstück 20 beim Einführen in die Austrittsöffnung 16 stößt. Der Vorsprung 70 hat mehrere rillenförmige, sich axial erstreckende Ausnehmungen 72, und ein sich zu seiner Mittelachse hin verjüngendes Ende 74. Gemäß Fig. 4 ist durch ein in die Austrittsöffnung 16 eingeführtes Anschlußstück 20 eines nicht gezeigten Auffangbehälters der Ventilkörper 28 vom Ventilsitz 30 in eine Öffnungsstellung zurückgedrängt worden. Das elastische Material der Auskleidung 44 gewährleistet für eine Vielzahl handelsüblicher Anschlußstücke 20 eine abdichtende Verbindung, die aufgrund der Klemmwirkung keiner zusätzlichen Fixierung bedarf. Beim Einführen des Anschlußstücks, das an seinem vorderen Ende in der Regel einen runden Öffnungsquerschnitt hat, ist mit Hilfe des sich verjüngenden Endes 74 des Vorsprungs 70 das Anschlußstück 20 in der Austrittsöffnung 16 zentriert. Die im Hohlraum 24b befindliche Flüssigkeit fließt mittels der rillenförmigen Ausnehmungen 72 in das Anschlußstück 20 ab. In Fig. 5 ist in einer Aufsicht auf den Vorsprung 70 die Anordnung der rillenförmigen Ausnehmungen 72 gezeigt. Der Vorsprung 70 hat einen zur Austrittsöffnung 16, die durch den anpassungsfähigen Öffnungsrand 18 gebildet ist, passenden runden Querschnitt. In Fig. 6 ist der Vorsprung 70 in einer weiteren Ausführungsform mit einem balligen Ende 76 dargestellt.

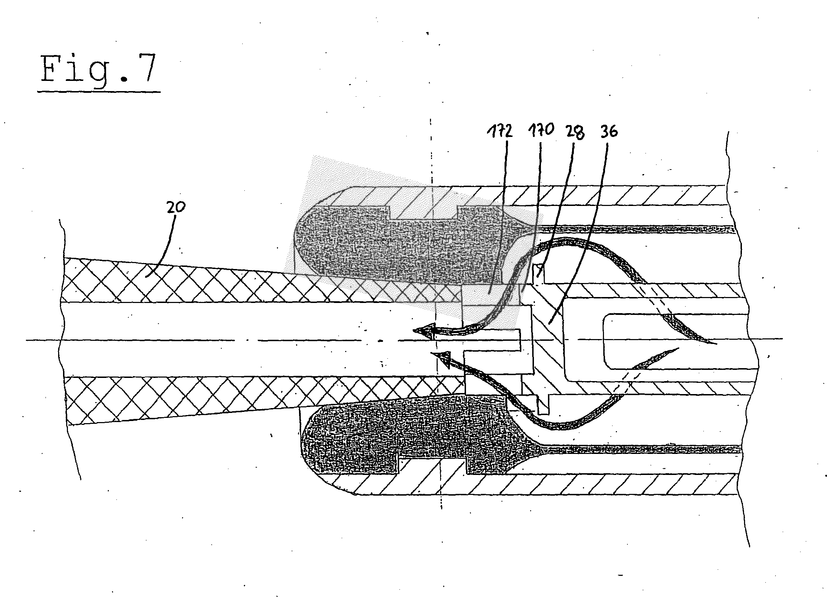

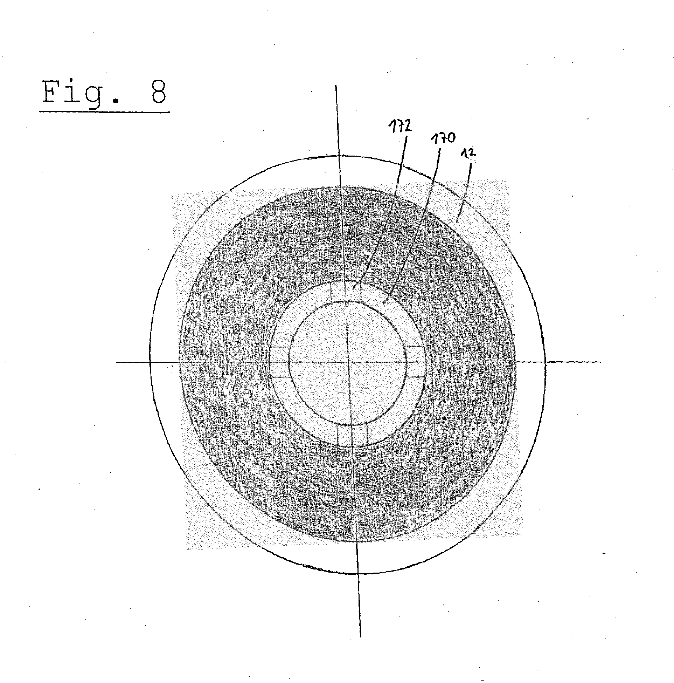

Ein Ausführungsbeispiel mit einer gegenüber Fig. 3 abgewandelten Ausbildung des Vorsprungs 70 zeigen Fig. 7 in einem Schnitt und Fig. 8 in einer Aufsicht. Ein sich vom Ventilkörper 28 durch die Dichtfläche des Ventilsitzes 30 hindurch zur Austrittsöffnung 16 erstreckender Vorsprung hat die Form einer ringförmig verlaufenden Wandung 170, die sich als eine Art Kragen auf der geschlossenen Stirnseite 36 des Ventilkörpers 28 befindet. Bei Einführen des Anschlußstücks 20 und Anstoßen gegen die Wandung 170 wird der Ventilkörper 28 zurückgedrängt. Der Flüssigkeitsabfluß erfolgt durch Aussparungen 172.

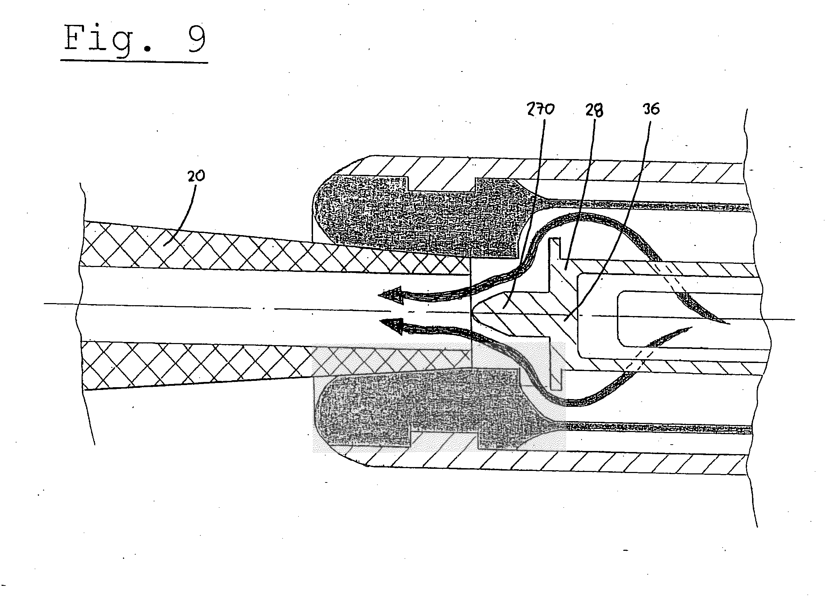

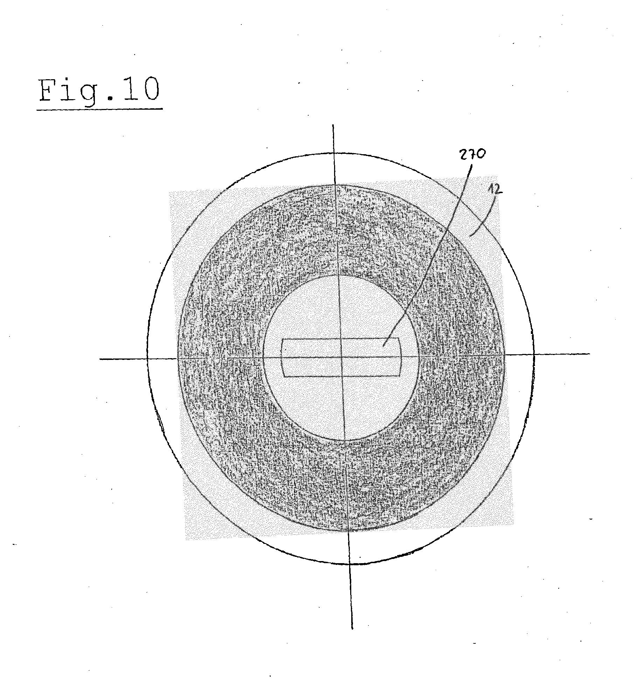

Eine weitere Modifikation des Vorsprungs 70 zeigen Fig. 9 in einem Schnitt und Fig. 10 in einer Aufsicht. Der dort dargestellte Vorsprung ist als ein sich vom Ventilkörper 28 durch die Dichtfläche des Ventilsitzes 30 hindurch zur Austrittsöffnung 16 erstreckender Steg 270 auf der geschlossenen Stirnseite 36 ausgebildet. Wiederum wird durch Einführen des Anschlußstücks 20 in die Austrittsöffnung 16 und Anstoßen gegen den Steg 270 der Ventilkörper 28 in eine Öffnungsstellung zurückgedrängt. Da der Steg 270 nur an zwei Stellen an dem Anschlußstück 20 anliegt, kann ein Abfluß erfolgen, ohne daß dazu weitere Aussparungen erforderlich sind.

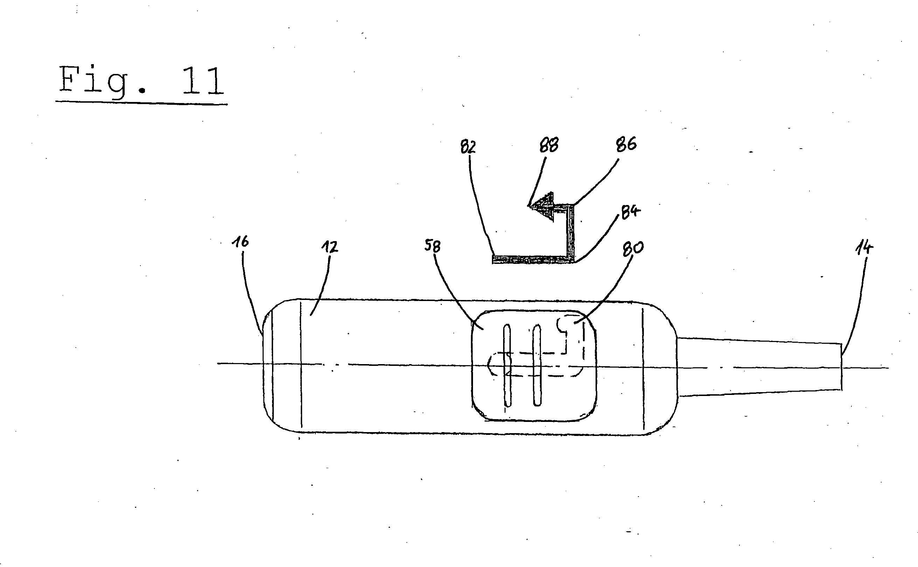

In Fig. 11 ist das erfindungsgemäße Katheterventil 10 mit Griffstück 58 in einer Seitenansicht mit einem an sich bekannten Schema zum Bewegungs- und Arretierungsvorgang gezeigt. Die Führungsnut 60 des Griffstücks bildet eine L-förmige Kulisse 80, in der es von einer ersten Position 82 axial in Richtung zur Eintrittsöffnung 14 in eine zweite Position 84, von dort quer zur Längsrichtung des Gehäuses 12 in eine dritte Position 86 und von dort ein wenig axial zurück in Richtung zur Austrittsöffnung in eine vierte Position 88 geführt wird. Wie in Kombination mit Fig. 1 ersichtlich ist, befindet sich das Katheterventil in der ersten Position 82 in geschlossener und in der zweiten und dritten Position 84 bzw. 86 in geöffneter Stellung. Auch in der vierten Position 88 des Griffstücks 58 ist das Ventil noch geöffnet. Durch die auf den Ventilkörper 28 ausgeübte Federkraft, die über die Rastnut 54 und den Vorsprung 56 auch auf das Griffstück 58 wirkt, ist eine unbeabsichtigte Verschiebung von der vierten Position 88 in die dritte Position 86 ausgeschlossen.

Wenn man ein Anschlußstück 20 eines Auffangbehälters in die Austrittsöffnung 16 einführt, wird mittels des Vorsprungs 70 der Ventilkörper 28 in eine Öffnungsstellung zurückgedrängt. Gleichzeitig wird auch das Griffstück 58, das mittels des Vorsprungs 56 über die Rastnut 54 mit dem Ventilkörper 28 in Eingriff steht, von der ersten Position 82 in die zweite Position 84 bewegt, und diese Bewegung kann dazu genutzt werden, daß auf der Außenseite des Gehäuses 12 ein Signalelement freigelegt wird, das in Fig. 11 nicht dargestellt ist. Auf diese Weise ist eine visuelle Kontrolle über den korrekten Anschluß des Auffangbehälters gegeben.

Bei manueller Öffnung des Katheterventils 10 ohne Anschluß eines Auffangbehälters erfolgt das Zurückdrängen des Ventilkörpers 28 weg vom Ventilsitz 30 in eine Öffnungsstellung mittels des Griffstücks 58. Dazu wird das Griffstück 58 von der ersten Position 82 in die zweite Position 84 geschoben. Falls gewünscht, kann zur dauerhaften Öffnung das Griffstück 58 weiter in die dritte Position 86 geschoben werden, aus der es selbsttätig in die vierte Position 88 gelangt. Für die Arretierung des Griffstücks 58 können selbstverständlich auch andere Mechanismen eingesetzt werden.

Varianten im Sinne der Ansprüche sind insbesondere auch im Bereich der Eintrittsöffnung 14 und der Austrittsöffnung 16 möglich. Wenn beispielsweise ein einheitliches Anschlußstück 20 der anzuschließenden Auffangbehälter auf dem Markt verfügbar ist, kann statt des adaptionsfähigen Öffnungsrands 18 eine an das einheitliche Anschlußstück 20 angepaßte Austrittsöffnung vorgesehen werden. Weitere Modifikationen lassen sich im Bereich der Hülse 26a, 26b realisieren, die beispielsweise auch einstückig hergestellt werden kann mit einer Rastnut für den Vorsprung 56 des Griffstücks 58.

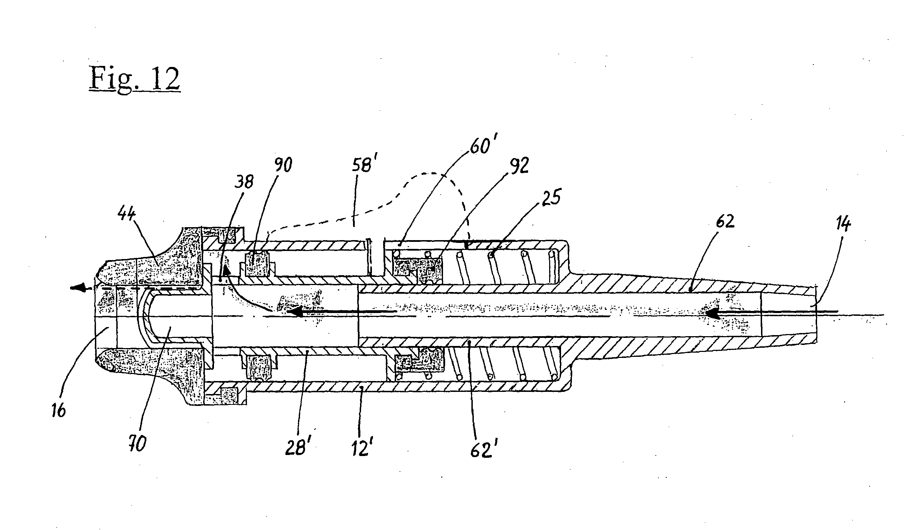

In Fig. 12 ist eine abgewandelte Ausführungsform des Katheterventils gezeigt. Hier ist das etwas abgewandelte Gehäuse mit 12' und der abgewandelte Ventilkörper mit 28' bezeichnet. Die Bezugszeichen 14, 16, 38, 44, 62 und 70 wurden wegen im Wesentlichen gleicher Funktion und Form der Teile unverändert übernommen.

Abweichend von der zuerst beschriebenen Ausführungsform kommen anstelle der Hülse 26a, 26b gleitende Dichtungen 90, 92 zwischen dem Ventilkörper und dem Gehäuse zur Anwendung. Die in Strömungsrichtung hintere, ringförmige Dichtung 90 sitzt in Strömungsrichtung axial unmittelbar vor den seitlichen Öffnungen 38 des Ventilkörpers 28' auf dessen äußerer Umfangsfläche und dichtet gegen die Innenfläche der Gehäusewand 12'. Die in Strömungsrichtung vordere Dichtung 92 sitzt auf dem vorderen Ende des Ventilkörpers 28' und dichtet gegen die äußere Umfangsfläche eines Kanals 62', der eine Fortsetzung des Eintrittskanals 62 in den von der Gehäusewand 12' umgebenen Innenraum des Ventils bildet. Der Kanal 62' ist vorzugsweise einstückig mit der Gehäusewand 12' und dem Eintrittskanal 62 geformt. Der Ventilkörper 28' wird durch eine Druckfeder 25, die in dem Ringraum zwischen dem Kanal 62' und der Gehäusewand 12' aufgenommen ist, in Schließrichtung des Ventils vorbelastet.

Ein Griffstück zur manuellen Betätigung des Katheterventils ist bei 58' angedeutet. Es ist zwischen den beiden Dichtungsringen 90, 92 fest mit dem Ventilkörper 28' verbunden oder einstückig mit diesem geformt und ragt durch ein Loch 60' oder eine Führung 60 gemäß Fig. 1 durch die Gehäusewand 12' nach außen, wobei das Loch 60' beziehungsweise die Führungsnut 60 eine solche axiale Länge hat, daß sich der Ventilkörper 28' zusammen mit dem daran angebrachten Griffstück 58' relativ zum Gehäuse 12' z. B. 10 mm aus der geschlossenen Ventilstellung mit Bezug auf Fig. 12 nach rechts in eine Öffnungsstellung verschieben läßt.

## Patentansprüche

1. Katheterventil mit einem Gehäuse (12), das eine an einen Urinableitungskatheter anschließbar Eintrittsöffnung (14), eine mit einem Anschlußstück (20) eines Auffangbehälters (22) dicht verbindbare Austrittsöffnung (16) und einen Ventilsitz (30) aufweist, der mit einem in Schließrichtung federnd vorbelasteten, axial verschieblichen Ventilkörper (28) zusammenwirkt, welcher mittels eines mit ihm in Eingriff stehenden Griffstücks (58) manuell in Öffnungsrichtung verschiebbar ist **dadurch gekennzeichnet, dass** der Ventilkörper einen sich durch die Dichtfläche des Ventilsitzes (30) hindurch zur Austrittsöffnung (16) erstreckenden Vorsprung (70) hat, wobei der Vorsprung (70) des Ventilkörpers (28) im Querschnitt um so viel kleiner als der Querschnitt der Austrittsöffnung (16) ist, dass ein axialer Strömungskanal in das Anschlußstück (20) frei bleibt, aber ausreichend groß, dass das in die Austrittsöffnung (16) einführbare Anschlußstück (20) stellenweise gegen den Vorsprung (70) stößt und dadurch der Ventilkörper (28) vom Ventilsitz (30) weg in eine Öffnungsstellung zurückdrängbar ist.

2. Katheterventil nach Anspruch 1, **dadurch gekennzeichnet, daß** die Austrittsöffnung (16) durch eine am hinteren Ende der Außenwand des Gehäuses (12) befestigte, aus anpassungsfähigem, elastischem Material bestehende, ringförmige Auskleidung (44) zur dichten Verbindung mit Anschlußstücken (20) unterschiedlicher Form oder Größe gebildet ist.

3. Katheterventil nach Anspruch 1 oder 2, **dadurch gekennzeichnet, daß** der Ventilkörper (28) ein länglicher, in axialer Wichtung verschieblicher Hohlkörper ist, dessen auf Seiten der Eintrittsöffnung (14) gelegene Stirnseite (34) offen ist, dessen andere Stirnseite (36) geschlossen ist und mit dem Ventilsitz (30) zusammenwirkt und der in Strömungsrichtung vor dem Ventilsitz (30) seitliche Öffnungen (38) hat.

4. Katheterventil nach Anspruch 3, **dadurch gekennzeichnet, daß** der Ventilkörper (28) axial beweglich in einem Hohlraum (24b) gehalten ist, der Teil des Strömungskanal durch das Gehäuse (12) ist und durch eine Hülse (26a, 26b) aus elastischem Material gebildet ist, die sich innerhalb des Gehäuses (12) erstreckt und an dessen Enden in dichter Verbindung mit der Ein- (14) und Austrittsöffnung (16) befestigt ist.

5. Katheterventil nach Anspruch 3 oder 4, **dadurch gekennzeichnet, daß** die Hülse (26a, 26b) axial geteilt ist und einen Kompressionsbalg (26a), der sich zwischen der Eintrittsöffnung (14) und einer äußeren Rastnut (54) des Ventilkörpers (28) erstreckt, sowie eine Dichtungstülle (26b) aufweist, die sich zwischen der Rastnut (54) und dem Ventilsitz (30) oder der Austrittsöffnung (16) ersteckt, wobei die federnde Vorbelastung des Ventilkörpers (28) durch den Kompressionsbalg (26a) und/oder durch die Dichtungstülle (26b) erzeugtt ist.

6. Katheterventil nach Anspruch 31, **dadurch gekennzeichnet, daß** der hohlzylindrische Ventilkörper (28') jeweils mittels gleitender Dichtungen (90, 92) axial verschieblich an der Innenfläche der zylindrischen Gehäusewand (12') und an der zylindrischen Außenfläche eines sich axial von der Eintrittsöffnung (14) in den von der Gehäusewand (12') umgrenzten inneren Hohlraum hinein erstreckenden Kanals (62') geführt und von einer in dem Ringraum zwischen der Gehäusewand(12') und dem Kanal (62') aufgenommenen Feder (25') in Schließrichtung vorbelasten ist.

7. Katheterventil nach Anspruch 6, **dadurch gekennzeichnet, daß** die Dichtung (90) zwischen dem Ventilkörper (28') und der Gehäusewand (12') mit Bezug auf die Strömungsrichtung von den seitlichen Öffnungen (38) und die Dichtung (92) zwischen dem sich an die Eintrittsöffnung (14) anschließenden Kanal (62') und dem Ventilkörper (28) an dessen in Strömungsrichtung vorderen Ende anbeordnet ist, und daß das mit dem Ventilkörper (28') verbundene Griffstück (58') im axialen Bereich zwischen den beiden ringförmigen Dichtungen (90, 92) durch ein Loch (60') oder eine Führungsnut (60) in der Gehäusewand (12') nach außen ragt.

8. Kathetarventil nach einem der Ansprüche 1 bis 7, **dadurch gekennzeichnet, daß** durch die Bewegung des Griffstücks (58, 58') bei der Verschiebung des Ventilkörpers (28, 28') mittels des Anschlußstücks (20) in eine Öffnungsstellung auf der Außenseite des Gehäuses (12, 12') ein Signalelement freilegbar ist.

9. Katheterventil nach einem der Ansprüche 1 bis 8, **dadurch gekennzeichnet, daß** das Griffstück (58) in der Öffnungsstellung des Ventils arretierbar ist.

## Claims

1. Catheter valve having a housing (12) that comprises an entry opening (14) that can be connected to a urine drain catheter, and an exit opening (16) that can be connected, in tight manner, to a connector part (20) of a collection container (22), and a valve seat (30) that acts in concert with a valve body (28) that is preloaded in closing direction in spring-like manner and is axially shiftable, and can be shifted manually in the opening direction by means of a handle part (58) engaging it, **characterised in that** the valve body has a projection (70) that extends through the sealing surface of the valve seat (30) to the exit opening (16), whereby the projection (70) of the valve body (28) has a cross-section that is smaller, to an appropriate extent, than the cross-section of the exit opening (16) such that an axial flow channel into the connector part (20) remains free, but sufficiently large such that the connecting part (20) that can be inserted into the exit opening (16) hits, in some places, against the projection (70) and that, by this means, the valve body (28) can be pushed away from the valve seat (30) into an opening position.

2. Catheter valve according to claim 1, **characterised in that** the exit opening (16) is formed by a ring-shaped lining (44), which is attached to the back end of the outer wall of the housing (12), consists of adaptable, elastic material, and is intended to tightly connect to connector parts (20) of different shape or size.

3. Catheter valve according to claim 1 or 2, **characterised in that** the valve body (28) is an elongate hollow body that can be shifted in axial direction and whose end face (34) situated on the sides of the entry opening (14) is open, whose other end face (36) is closed and acts in concert with the valve seat (30) and which has lateral openings (38) upstream of the valve seat (30) with respect to the direction of flow.

4. Catheter valve according to claim 3, **characterised in that** the valve body (28) is held, in axially mobile manner, in a hollow space (24b) that is part of the flow channel through the housing (12) and is formed by a sleeve (26a, 26b) made of elastic material that extends inside the housing (12) and is attached to the ends thereof in tight connection to the entry opening (14) and exit opening (16).

5. Catheter valve according to claim 3 or 4, **characterised in that** the sleeve (26a, 26b) is parted axially and comprises a compression bellows (26a), which extends between the entry opening (14) and an outer snap-in groove (54) of the valve body (28), as well as a sealing grommet (26b), which extends between the snap-in groove (54) and the valve seat (30) or the exit opening (16), whereby the spring-like preload of the valve body (28) is generated by the compression bellows (26a) and/or by the sealing grommet (26b).

6. Catheter valve according to claim 3, **characterised in that** the hollow cylindrical valve body (28') is guided, in each case, by means of gliding seals (90, 92), in axially shiftable manner, on the inner surface of the cylindrical housing wall (12') and on the cylindrical outer surface of a channel (62') that extends axially from the entry opening (14) into the inner hollow space that is bounded by the housing wall (12') and is preloaded in the closing direction by a spring (25') that is accommodated in the ring space between the housing wall (12') and the channel (62').

7. Catheter valve according to claim 6, **characterised in that** the seal (90) between the valve body (28') and the housing wall (12') is arranged upstream, with respect to the direction of flow, of the lateral openings (38) and the seal (92) between the channel (62') adjacent to the entry opening (14) and the valve body (28) is arranged on the front end thereof in the direction of flow, and **in that** the handle part (58') connected to the valve body (28') extends, in the axial region between the two ring-shaped seals (90, 92), through a hole (60') or a guide groove (60) in the housing wall (12') towards the outside.

8. Catheter valve according to any one of the claims 1 to 7, **characterised in that** a signal element can be exposed by the motion of the handle part (58, 58') upon the shifting of the valve body (28, 28') by means of the connector part (20) into an opening position on the outside of the housing (12, 12').

9. Catheter valve according to any one of the claims 1 to 8, **characterised in that** the handle part (58) can be locked in the opening position of the valve.

## Revendications

1. Valve pour cathéter comprenant un boîtier (12), qui présente un orifice d'entrée (14) pouvant être raccordé à un cathéter urinaire, un orifice de sortie (16) pouvant être relié de manière étanche à une pièce de raccordement (20) d'un récipient collecteur (22) et un siège de valve (30), qui coopère avec un corps de valve (28) mobile axialement, précontraint de manière résiliente dans le sens de fermeture, qui peut être déplacé manuellement dans le sens d'ouverture au moyen d'un élément de poignée (58) se trouvant en prise avec lui, **caractérisée en ce que**, le corps de valve possède une saillie (70) s'étendant à travers la surface d'étanchéité du siège de valve (30) jusqu'à l'orifice de sortie (16), la section transversale de la saillie (70) du corps de valve (28) étant plus petite que la section transversale de l'orifice de sortie (16) dans une telle mesure qu'un conduit d'écoulement axial allant dans la pièce de raccordement (20) reste libre, mais suffisamment grande pour que la pièce de raccordement (20) pouvant être introduite dans l'orifice de sortie (16) heurte par endroits la saillie (70) et que le corps de valve (28) puisse être ainsi repoussé depuis le siège de valve (30) dans une position d'ouverture.

2. Valve pour cathéter selon la revendication 1, **caractérisée en ce que** l'orifice de sortie (16) est formé par un revêtement (44) annulaire, composé de matière élastique adaptable, fixé sur l'extrémité arrière de la paroi extérieure du boîtier (12) pour la connexion étanche à des pièces de raccordement (20) de formes ou de tailles différentes.

3. Valve pour cathéter selon la revendication 1 ou 2, **caractérisée en ce que** le corps de valve (28) est un corps creux allongé mobile dans le sens axial, dont la face frontale (34) située du côté de l'orifice d'entrée (14) est ouverte, dont l'autre face frontale (36) est fermée et coopère avec le siège de valve (30) et qui possède des orifices latéraux (38) en amont du siège de valve (30) dans le sens d'écoulement.

4. Valve pour cathéter selon la revendication 3, **caractérisée en ce que** le corps de valve (28) est maintenu mobile axialement dans une cavité (24b), qui fait partie du conduit d'écoulement à travers le boîtier (12) et qui est formée par un manchon (26a, 26b) en matière élastique, qui s'étend à l'intérieur du boîtier (12) et est fixé aux extrémités de celui-ci en connexion étanche avec l'orifice d'entrée (14) et de sortie (16).

5. Valve pour cathéter selon la revendication 3 ou 4, **caractérisée en ce que** le manchon (26a, 26b) est divisé axialement et présente un soufflet de compression (26a) qui s'étend entre l'orifice d'entrée (14) et une rainure d'arrêt (54) extérieure du corps de valve (23), ainsi qu'une douille d'étanchéité (26b) qui s'étend entre la rainure d'arrêt (54) et le siège de valve (30) ou l'orifice de sortie (16), la précontrainte résiliente du corps de valve (28) étant générée par le soufflet de compression (26a) et/ou la douille d'étanchéité (26b).

6. Valve pour cathéter selon la revendication 3, **caractérisée en ce que** le corps de valve (28') cylindrique est guidé respectivement au moyen de joints d'étanchéité (90, 92) coulissants de manière mobile axialement sur la surface intérieure de la paroi de boîtier (12') cylindrique et sur la surface extérieure cylindrique d'un conduit (62') s'étendant axialement dans la cavité intérieure entourée par la paroi de boîtier (12') depuis l'orifice d'entrée (14) et est précontraint dans le sens de fermeture par un ressort (25') reçu dans l'espace annulaire entre la paroi de boîtier (12') et le conduit (62').

7. Valve pour cathéter selon la revendication 6, **caractérisée en ce que** le joint d'étanchéité (90) entre le corps de valve (28') et la paroi de boîtier (12') est agencé en amont des orifices latéraux (38) par rapport au sens d'écoulement et le joint d'étanchéité (92) entre le conduit (62') raccordé à l'orifice d'entrée (14) et le corps de valve (28) est agencé sur l'extrémité avant de ce dernier dans le sens d'écoulement, et **en ce que** l'élément de poignée (58') relié au corps de valve (28') dépasse vers l'extérieur dans la zone axiale entre les deux joints d'étanchéité (90, 92) annulaires à travers un trou (60') ou une rainure de guidage (60) dans la paroi de boîtier (12').

8. Valve pour cathéter selon l'une des revendications 1 à 7, **caractérisée en ce que**, par le mouvement de l'élément de poignée (58, 58') lors du déplacement du corps de valve (28, 28') dans une position d'ouverture au moyen de la pièce de raccordement (20), un élément de signal peut être découvert sur la face extérieure du boîtier (12, 12').

9. Valve pour cathéter selon l'une des revendications 1 à 8, **caractérisée en ce que** l'élément de poignée (58) peut être bloqué dans la position d'ouverture de la valve.
